# EUROPEAN PATENT APPLICATION

(11) **EP 3 306 296 A1**
(43) Date of publication of application: **11.04.2018**
(21) Application number: 17175438.5
(22) Date of filing: 12.06.2017
(51) Int. Cl.: G01M 17/007, G01M 15/02, G01M 15/04, G01M 15/10

(54) **VEHICLE TEST SYSTEM, METHOD FOR MOVING TEST VEHICLE AND SIMULATED WHEEL**

(30) Priority: 05.10.2016 JP 2016197159
(71) Applicant: Horiba, Ltd., Kyoto-shi, Kyoto 601-8510 (JP)
(72) Inventor: MIZUTA, Masao, Kyoto, 601-8510 (JP)
(74) Representative: Müller Hoffmann & Partner

(57) **Abstract**

In order to make it possible to move a test vehicle (V) easily by using a big power in case of moving the test vehicle (V) accompanying a vehicle test by the use of a simulated wheel 10, this vehicle test system comprises a dynamometer 20 that gives a load to the test vehicle (V), a simulated wheel 10 having a rotary connection part 11 to one side of which the dynamometer 20 is connected and to the other side of which a vehicle shaft (S) of the test vehicle (V) is connected in case of testing the test vehicle (V) and that does not transmit a rotation of the vehicle shaft (S) to a tire, and a wheel for driving 40 that is connected to the rotary connection part 11 from which the dynamometer 20 is dismounted and to which the rotation of the vehicle shaft (S) is transmitted in case of moving the test vehicle (V).

## Description

### FIELD OF THE ART

This invention relates to a vehicle test system, a method for moving a test vehicle and a simulated wheel.

### BACKGROUND ART

Conventionally, as a vehicle test system to conduct a test by giving a load from a dynamometer to a vehicle shaft of the test vehicle, in order to simulate a state wherein an actual vehicle runs, there is a vehicle test system that can conduct a test in a state wherein a suspension or a damper is in operation by connecting a simulated wheel to a vehicle shaft of the test vehicle.

The simulated wheel is so configured that the connection part to which the vehicle shaft is connected rotates without rotating a tire. With this arrangement, in case of moving the test vehicle, even though the simulated wheel is connected to the vehicle shaft, the power transmitted from an engine to the vehicle shaft is not transmitted to the tire so that it is not possible to move the test vehicle.

As a method for moving the test vehicle, for example, before and after the test while conducting the vehicle test by making use of the simulated wheel, there is a method, as disclosed in the patent document 1, wherein the test vehicle is towed together with the simulated wheel by a wrecker.

In addition, another method is, as shown in the patent document 2, to make use of a bearing to which a vehicle shaft of a test vehicle is connected and a simulated wheel having a tire mounting part to which a tire is mounted and to move the test vehicle by connecting the bearing and the tire mounting part by a key at a time of moving the test vehicle and by transmitting a power from an engine to the vehicle shaft to the tire mounting part so as to rotate the simulated wheel.

However, the method described in the patent document 1 takes time to move the test vehicle. In addition, in accordance with the method described in the patent document 2, a mechanical strength of the key is week so that it is not possible to increase power from the engine although it is easier to move the test vehicle compared with the method described in the patent document 1 wherein the test vehicle is towed by the wrecker. As a result of this, according to the method described in the patent document 2, the test vehicle is moved at low speed and it is difficult to elongate the distance of moving the test vehicle.

### PRIORART DOCUMENTS

### PATENT DOCUMENTS

Patent document 1: Japanese Unexamined Utility Model Application Publication No. 63-165550
Patent document 2: Japanese Unexamined Patent Application Publication No. 2010-121988

### DISCLOSURE OF THE INVENTION

### PROBLEMS TO BE SOLVED BY THE INVENTION

The present claimed invention intends to solve all of the problems and a main object of this invention is to make it possible to move a test vehicle easily by using a big power in case of moving the test vehicle accompanying a vehicle test by the use of a simulated wheel.

### MEANS TO SOLVE THE PROBLEMS

More specifically, the vehicle test system in accordance with this invention comprises a dynamometer that gives a load to a test vehicle, a simulated wheel having a rotary connection part to one side of which the dynamometer is connected and to the other side of which a vehicle shaft of the test vehicle is connected in case of testing the test vehicle and that does not transmit a rotation of the vehicle shaft to a tire and a wheel for driving that is connected to the rotary connection part from which the dynamometer is dismounted and to which the rotation of the vehicle shaft is transmitted in case of moving the test vehicle.

In accordance with the vehicle test system having this arrangement, it is possible to transmit the rotation of the vehicle shaft to the wheel for driving without transmitting the rotation of the vehicle shaft to a tire just by connecting the wheel for driving to the rotary connection part from which the dynamometer is dismounted. With this arrangement, since the wheel for driving rotates together with the vehicle shaft, it is possible to move the test vehicle easily without using a wrecker or the like.

In addition, since the rotary connection part is a part to be connected to the dynamometer and the mechanical strength of the rotary connection part is relatively strong so that the rotary connection part can withstand a power mechanically enough even though the power transmitted to the wheel for driving is increased, it becomes possible to move the test vehicle at a higher speed and for a longer distance compared with a conventional system.

As a concrete arrangement of the wheel for driving represented is the wheel for driving that comprises a wheel to which the rotary connection part is connected and a driving tire that transmits a rotation of the wheel to a road surface.

If the load of the moving test vehicle is to be supported by the wheel for driving alone, it is necessary to use the high load-resistant wheel for driving such that a width dimension of the wheel for driving is large.

In case of moving the test vehicle, it is preferable that both the simulated wheel and the wheel for driving are grounded on a road surface, namely both the simulated wheel and the wheel for driving support the load of the test vehicle.

In accordance with this arrangement, since the load of the moving test vehicle can be supported by both the simulated wheel and the wheel for driving, it is possible to use the low load-resistant wheel for driving that is easy to handle such that a width dimension of the wheel for driving is small.

In case that both the simulated wheel and the wheel for driving are grounded, it is preferable that the load of the test vehicle supported by the simulated wheel is bigger than the load of the test vehicle supported by the wheel for driving.

Concretely, in order to make it possible to support the load of the test vehicle mainly by the simulated wheel, it is preferable that an outer diameter of the wheel for driving is equal to or smaller than an outer diameter of the simulated wheel.

In case that the outer diameter of the wheel for driving is smaller than the outer diameter of the simulated wheel, the outer diameter of the wheel for driving may be so set to ground because the test vehicle supported by the simulated wheel sinks due to the vehicle's own weight.

It is preferable to further comprise a connection member that locates between the wheel for driving and the rotary connection part and that connects the wheel for driving and the rotary connection part and that the wheel for driving and the connection member are spline-connected.

In accordance with this arrangement, even though the vehicle shaft moves in the axial direction due to vibration of the test vehicle or the like, it is possible to transmit the rotation of the vehicle shaft to the wheel for driving while releasing displacement generating in the axial direction at a portion where the wheel for driving and the connection member are connected.

As a more concrete embodiment of the vehicle test system represented is a system further comprising an exhaust gas analyzer that analyzes an exhaust gas of the test vehicle.

In addition, the simulated wheel in accordance with this invention has a rotary connection part to one side of which a dynamometer is connected and to the other side of which a vehicle shaft of a test vehicle is connected in case of testing the test vehicle and that does not transmit a rotation of the vehicle shaft to a tire, and is characterized by that a wheel for driving to which a rotation of the vehicle shaft is transmitted is connected to the rotary connection part from which the dynamometer is dismounted in case of moving the test vehicle.

Furthermore, a method for moving a test vehicle in accordance with this invention is a method for moving the test vehicle accompanying a vehicle test that is conducted by the use of a dynamometer that gives a load to the test vehicle and a simulated wheel having a rotary connection part to one side of which the dynamometer is connected and to the other side of which a vehicle shaft of the test vehicle is connected in case of testing the test vehicle and that does not transmit a rotation of the vehicle shaft to a tire, and is characterized by that a wheel for driving is connected to the rotary connection part from which the dynamometer is dismounted, and the test vehicle is moved by transmitting the rotation of the vehicle shaft to the wheel for driving.

In accordance with the method for moving the test vehicle of the unit for the test vehicle, it is possible to obtain the same operation and effect as that of the above-mentioned vehicle test system.

In addition, for the above-mentioned method for moving the test vehicle, it is preferable that at least one of an air pressure of the wheel for driving and the air pressure of the simulated wheel is adjusted after the wheel for driving is connected to the rotary connection part and before the test vehicle is moved.

In accordance with this arrangement, it is possible to adjust a ratio of the load of the test vehicle applied to the wheel for driving and the simulated wheel.

### EFFECT OF THE INVENTION

In accordance with this invention having the above-mentioned arrangement, it is possible to move the test vehicle easily by the use of a big power in case of moving the test vehicle accompanying the vehicle test using the simulated wheel.

### BRIEF DESCRIPTION OF THE DRAWINGS

Fig. 1 is a pattern diagram showing a configuration of the vehicle test system at a testing time in accordance with this embodiment.
Fig. 2 is a pattern diagram showing a configuration of the vehicle test system at a time of moving a test vehicle in accordance with this embodiment.
Fig. 3 is a pattern diagram showing a configuration of a wheel for driving in accordance with this embodiment.

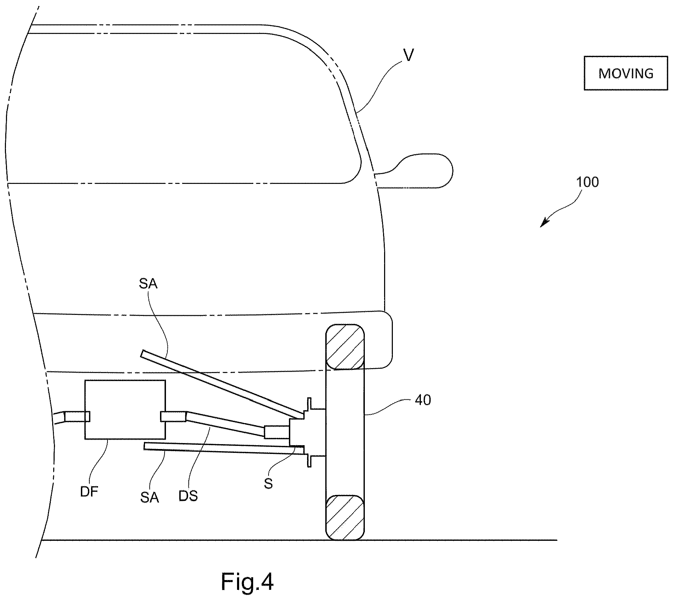

Fig. 4 is a pattern diagram showing a configuration of the vehicle test system in accordance with other embodiment.
Fig. 5 is a pattern diagram showing a configuration of the wheel for driving in accordance with the other embodiment.

### BEST MODES OF EMBODYING THE INVENTION

One embodiment of a vehicle test system in accordance with this invention will be explained with reference to drawings.

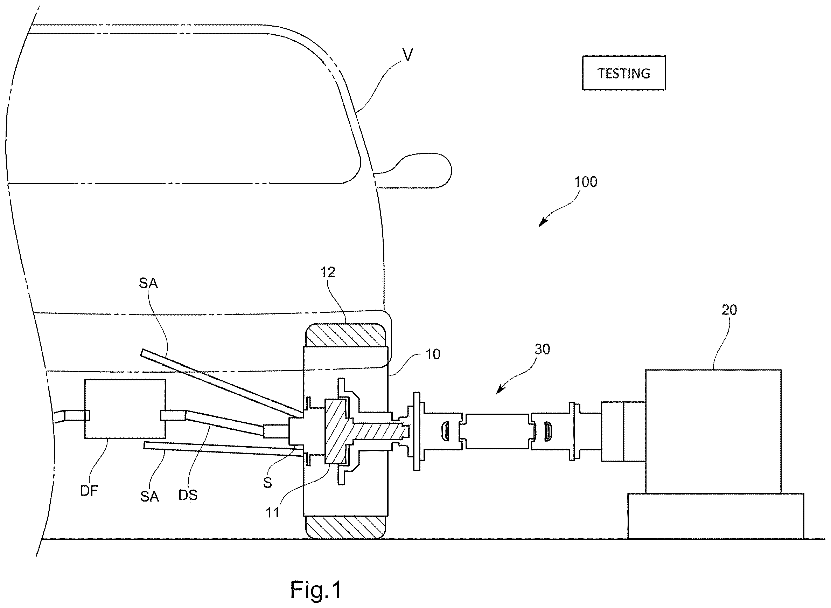

The vehicle test system 100 in accordance with this embodiment is used for testing, for example, a performance of an engine or a transmission of a test vehicle (V), and concretely as shown in Fig. 1, comprises a simulated wheel 10 to which a vehicle shaft (S) of the test vehicle (V) is connected, a dynamometer 20 that is connected to the simulated wheel 10 at an opposite side to the vehicle shaft (S), a torque sensor, not shown in drawings, that detects torque applied to the vehicle shaft (S) and an exhaust gas analyzer, not shown in drawings, that analyzes an exhaust gas of the test vehicle (V).

The test vehicle (V) may be two-wheel drive such as FF or FR, or may be four-wheel drive.

The simulated wheel 10 has, as shown in Fig. 1, a rotary connection part 11 to which the vehicle shaft (S) is connected and that rotatably supports the vehicle shaft (S) and a tire 12 that keeps a static state although the rotary connection part 11 rotates, and is so arranged that the rotary connection part 11 rotates without rotating the tire 12 although the vehicle shaft (S) rotates. The simulated wheel 10 in accordance with this embodiment makes use of so-called a free wheel hub as being a hub that separates a driving force from the vehicle shaft (S). A bearing, not shown in drawings, (for example, a ball bearing) is arranged between the rotary connection part 11 and the tire 12 so as not to transmit the rotation of the vehicle shaft (S) to the tire 12.

In order to simulate a running state of an actual vehicle, as shown in Fig. 1, a drive shaft (DS) connected to a differential (DF), a suspension arm (SA) (an upper arm or a lower arm), and a dumper or a spring, not shown in drawings, are mounted on the simulated wheel 10 and a test can be conducted in a state wherein these mounted constituting members are in operation.

The dynamometer 20 gives a load to an engine or the vehicle shaft (S), and it is so set that the load from the dynamometer 20 given to the vehicle shaft (20) is equal to the load applied to the vehicle shaft (S) at a time of actual running. The dynamometer 20 is connected to the rotary connection part 11 through a joint 30. With this arrangement, the load of the dynamometer 20 is transmitted to the vehicle shaft (S) through the joint 30 and the rotary connection part 11.

In this embodiment, a constant velocity joint is used as the joint 30. The constant velocity joint 30 is a joint used for transmitting the rotational movement. Even though an input shaft is at an angle to an output shaft, torque is transmitted by rotating both shafts at the same speed.

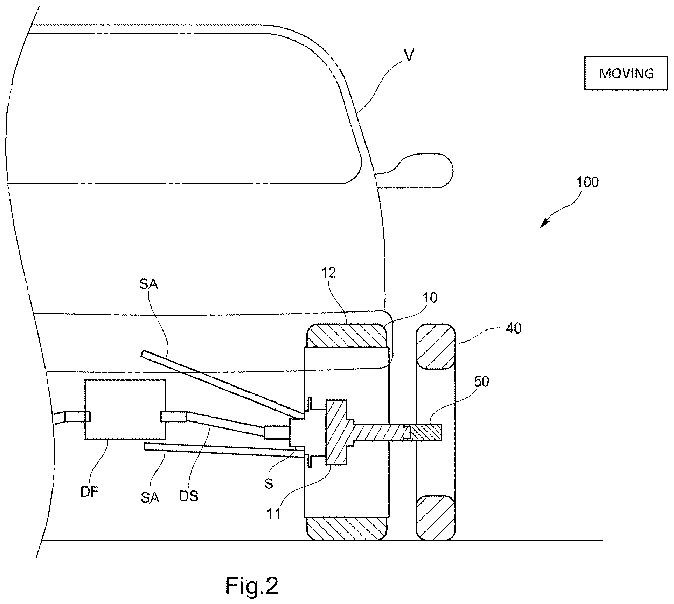

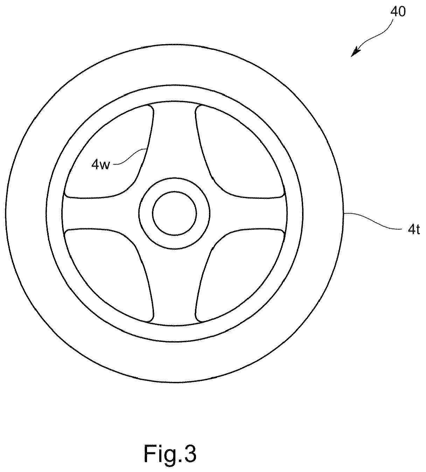

As shown in Fig. 2, the vehicle test system 100 of this embodiment further comprises a wheel for driving 40 that is connected to an opposite side to the vehicle shaft (S) of the rotary connection part 11 from which the dynamometer 20 is dismounted in case of moving the test vehicle (V) accompanying the vehicle test, namely before and after the vehicle test. In case of moving the test vehicle (V), the wheel for driving 40 may be connected to all of the four vehicle shafts (S) locating front, rear, right and left, or in case that the test vehicle (V) is a two-wheel drive vehicle such as front engine front drive or front engine rear drive, the wheel for driving 40 may be connected to only two vehicle shafts (S) locating front or rear to which the power of the engine is transmitted. As shown in Fig. 3, the wheel for driving 40 comprises a wheel 4w to which the rotary connection part 11 of the simulated wheel 10 is connected and a driving tire 4t that transmits a rotation of the wheel 4w to a road surface.

Concretely, in case of comparing an outer diameter of the wheel for driving 40 with an outer diameter of the simulated wheel 10 in a state that no load is applied, the outer diameter of the wheel for driving 40 is equal to or smaller than the outer diameter of the simulated wheel 10. In addition, a width of the wheel for driving 40 is, as shown in Fig. 2, smaller than a width of the simulated wheel 10. It is acceptable that the width of the wheel for driving 40 is equal to or bigger than the width of the simulated wheel 10.

The wheel for driving 40 is connected to the rotary connection part 11 through a connection member 50. In this embodiment, after the joint 30 and the dynamometer 20 are dismounted from the rotary connection part 11, one end of the connection member 50 is connected to the rotary connection part 11 and the wheel for driving 40 is connected to the other end of the connection member 50. At this time, the test vehicle (V) may be jacked-up in order to facilitate the operation of connecting the rotary connecting part 11 and the wheel for driving 40. After the wheel for driving 40 is connected to the other end of the connection member 50, the wheel for driving 40 is fixed to the other end of the connection member 50 by the use of a nut or a retaining plate, not shown in drawings, in order to prevent the wheel for driving 40 from being dismounted. The other end of the connection member 50 and the wheel for driving 40 are spline-connected. In this embodiment, one connection member 50 is used, however, multiple connection members 50 may be used to connect the wheel for driving 40 and the rotary connection part 11. The spline connection is a connecting method to transmit the driving force while releasing a displacement generating in an axial direction.

The outer diameter of the wheel for driving 40 in this embodiment is expandable, and the wheel for driving 40 is connected to the rotary connection part 11 in a state where the outer diameter of the wheel for driving 40 is smaller than the outer diameter of the simulated wheel 10. After the wheel for driving 40 is connected, the outer diameter of the wheel for driving 40 is increased so as to be a little bigger than the outer diameter of the simulated wheel 10 or generally equal to the outer diameter of the simulated wheel 10. With this arrangement, both the wheel for driving 40 and the simulated wheel 10 are grounded while the test vehicle (V) moves. The outer diameter of the wheel for driving 40 at a time of moving the test vehicle (V) is so set to transmit the power from the wheel for driving 40 to the ground.

A method for expanding or reducing the outer diameter of the wheel for driving 40 is represented by a method wherein air is taken into or taken out from the wheel for driving 40. With this method, the wheel for driving 40 is connected to the rotary connection part 11 in a state that the air is drawn out from the wheel for driving 40 to a certain degree and later the wheel for driving 40 is filled with the air.

Furthermore, prior to moving the test vehicle (V) after the wheel for driving 40 is connected to the rotary connection part 11, air pressure of the simulated wheel 10 and/or the wheel for driving 40 is adjusted by putting or discharging the air into or from one or both of the simulated wheel 10 and the wheel for driving 40 so that distribution of the load of the test vehicle (V) applied to the simulated wheel 10 and the wheel for driving 40 is adjusted.

In accordance with the vehicle test system 100 having the above-mentioned arrangement in accordance with this embodiment, the rotation of the vehicle shaft (S) is transmitted to the wheel for driving 40 so that the wheel for driving 40 rotates just by connecting the wheel for driving 40 to the rotary connection part 11 from which the dynamometer 20 is dismounted. As a result of this, it is possible to move the test vehicle (V) easily without using a wrecker or the like.

In addition, since the rotary connection part 11 to which the simulated wheel 10 is connected is a part to which the dynamometer 20 is connected so that mechanical strength of the rotary connection part 11 is relatively strong and the rotary connection part 11 is capable of mechanically enduring the power even though the power transmitted to the wheel for driving 40 is increased, it is possible to move the test vehicle (V) for a long distance at a high speed.

Furthermore, since the wheel for driving 40 and the connection member 50 are spline-connected, it is possible to transmit the rotation of the vehicle shaft (S) to the wheel for driving 40 while releasing displacement generating in the axial direction at a portion where the wheel for driving 40 and the connection member 50 are connected, even though the vehicle shaft (S) moves in the axial direction due to vibration of the test vehicle (V).

In addition, since both the simulated wheel 10 and the wheel for driving 40 are grounded on the road surface in case of moving the test vehicle (V), the load of the test vehicle (V) can be supported by both the simulated wheel 10 and the wheel for driving 40 so that it is possible to use the low load-resistant wheel for driving 40 that is easy to deal with. Furthermore, in case that the outer diameter of the wheel for driving 40 is smaller than the outer diameter of the simulated wheel 10, the simulated wheel 10 supports the load of the test vehicle (V) more than the wheel for driving 40 does.

The present claimed invention is not limited to the above-mentioned embodiment.

For example, the wheel for driving 40 is connected to the rotary connection part 11 of the simulated wheel 10 in the above-mentioned embodiment, however, the wheel for driving 40 may be connected to the vehicle shaft (S) from which the simulated wheel 10 is dismounted.

In addition, the wheel for driving 40 may be directly connected to the rotary connection part 11 by the use of a nut or a retaining plate, not shown in drawings, without using the connection member 50. The wheel for driving 40 may also be closely connected to the simulated wheel 10.

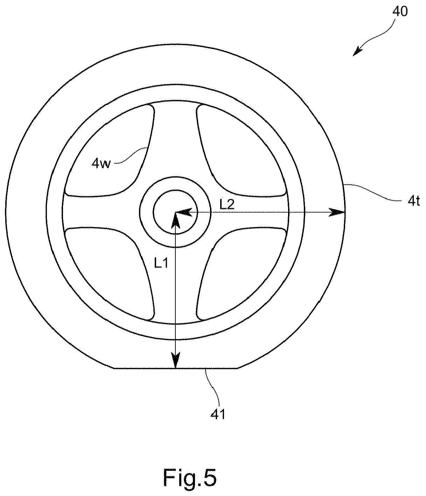

Furthermore, the wheel for driving 40 is connected to the rotary connection part 11 in a state of being deflated to a certain degree in the above-mentioned embodiment, however, as shown in Fig. 5, the wheel for driving 40 having a cutout part 41 formed on a part of an outer peripheral part may be used.

Concretely, the wheel for driving 40 having the cutout part 41 is so configured that a length L1 from a center of the wheel for driving 40 to the cut-out part 41 is smaller than the outer diameter of the simulated wheel 10 and a length L2 from the center of the wheel for driving 40 to the outer peripheral part excepting the cutout part 41 is bigger than or equal to the outer diameter of the simulated wheel 10. The cutout part 41 may be formed at multiple portions.

In accordance with this arrangement, it is possible to mount the wheel for driving 40 easily without inflating or deflating the wheel for driving 40 like in the above-mentioned embodiment by connecting the wheel for driving 40 to the rotary connection part 11 or the vehicle shaft (S) in a state wherein the cutout part 41 locates in the lower side of the wheel for driving 40.

The wheel for driving 40 may be in an oval shape having no cutout part 41.

In addition, the outer diameter of the wheel for driving 40 is expanded or reduced by inflating or deflating the wheel for driving 40 in the above-mentioned embodiment, however, the outer diameter of the wheel for driving 40 may be expanded or reduced by providing an elastic member such as a spring that is expandable in a radial direction inside of the wheel for driving 40.

Furthermore, the wheel for driving 40 may be a wheel whose outer diameter is a little smaller than the outer diameter of the simulated wheel 10. In this case, both the simulated wheel 10 and the wheel for driving 40 have only to be grounded at a time of moving the test vehicle (V) by deflating the simulated wheel 10 so as to reduce the outer diameter of the simulated wheel 10 after the wheel for driving 40 is mounted on the rotary connection part 11.

In the above-mentioned embodiment, it has been explained that the wheel for driving 40 whose outer diameter is equal to or smaller than the outer diameter of the simulated wheel 10 is used because it becomes easy to mount the wheel for driving 40 on the simulated wheel 10 and load capacity to support all load of the test vehicle (V) is not required, however, it shall not preclude to use the wheel for driving 40 whose outer diameter is bigger than the outer diameter of the simulate wheel 10. The wheel for driving 40 whose outer diameter is bigger than the outer diameter of the simulated wheel 10 may be used if the load capacity of the wheel for driving 40 is increased by contriving a method for mounting the wheel for driving 40 such as jacking-up the test vehicle (V).

In addition, it is a matter of course that the present claimed invention is not limited to the above-mentioned embodiment and may be variously modified without departing from a spirit of the invention.

### EXPLANATION OF CODES

- 100: vehicle test system
- V: test vehicle
- S: vehicle shaft
- 10: simulated wheel
- 11: rotary connection part
- 12: tire
- 20: dynamometer
- 30: joint
- 40: wheel for driving
- 50: connection member

## Claims

1. A vehicle test system comprising
a dynamometer that gives a load to a test vehicle,
a simulated wheel having a rotary connection part to one side of which the dynamometer is connected and to the other side of which a vehicle shaft of the test vehicle is connected in case of testing the test vehicle and that does not transmit a rotation of the vehicle shaft to a tire.
a wheel for driving that is connected to the rotary connection part from which the dynamometer is dismounted and to which the rotation of the vehicle shaft is transmitted in case of moving the test vehicle.

2. The vehicle test system described in claim 1, wherein
the wheel for driving comprises a wheel to which the rotary connection part is connected and a driving tire that transmits a rotation of the wheel to a road surface.

3. The vehicle test system described in claim 1, wherein
both the simulated wheel and the wheel for driving are grounded on a road surface in case of moving the test vehicle.

4. The vehicle test system described in claim 1,
an outer diameter of the wheel for driving is equal to or smaller than an outer diameter of the simulated wheel.

5. The vehicle test system described in claim 1,
both the simulated wheel and the wheel for driving support the load of the test vehicle in case of moving the test vehicle.

6. The vehicle test system described in claim 5,
the load of the test vehicle supported by the simulated wheel is bigger than the load of the test vehicle supported by the wheel for driving.

7. The vehicle test system described in claim 1, further comprising
a connection member that locates between the wheel for driving and the rotary connection part and that connects the wheel for driving and the rotary connection part in case of moving the test vehicle, wherein
the wheel for driving and the connection member are spline-connected.

8. The vehicle test system described in claim 1, and further comprising an exhaust gas analyzer that analyzes an exhaust gas of the test vehicle.

9. A simulated wheel having a rotary connection part to the other side of which a vehicle shaft of the test vehicle is connected and that does not transmit a rotation of the vehicle shaft to a tire, wherein
to one side of the rotary connection part of the simulated wheel, a dynamometer is connected in case of testing the test vehicle, and to the rotary connection part from which the dynamometer is dismounted, a wheel for driving to which a rotation of the vehicle shaft is transmitted is connected in case of moving the test vehicle.

10. Amethod for moving a test vehicle that is a method for moving the test vehicle accompanying a vehicle test that is conducted by the use of a dynamometer that gives a load to the test vehicle and a simulated wheel having a rotary connection part to one side of which the dynamometer is connected and to the other side of which a vehicle shaft of the test vehicle is connected in case of testing the test vehicle and that does not transmit a rotation of the vehicle shaft to a tire, wherein
a wheel for driving is connected to the rotary connection part from which the dynamometer is dismounted, and
the test vehicle is moved by transmitting the rotation of the vehicle shaft to the wheel for driving.

11. The method for moving the test vehicle described in claim 10, wherein
at least one of an air pressure of the wheel for driving and the air pressure of the simulated wheel is adjusted after the wheel for driving is connected to the rotary connection part and before the test vehicle is moved.
